# EUROPEAN PATENT SPECIFICATION

(11) **EP 2 895 256 B1**
(45) Date of publication and mention of the grant of the patent: **14.11.2018**
(21) Application number: 13762816.0
(22) Date of filing: 13.09.2013
(51) Int. Cl.: B01D 69/12, B01D 69/14, C07F 9/10, C12Q 1/00, G01N 27/333, G01N 33/543, G01N 27/327

(54) **ION FLUX BASED POTENTIOMETRIC SENSOR AND ITS USE**
IONENFLUSS BASIERTER POTENTIOMETRISCHER SENSOR UND DESSEN VERWENDUNG
CAPTEUR POTENTIOMÉTRIQUE BASÉ SUR UN FLUX IONIQUE ET SON UTILISATION

(30) Priority: 14.09.2012 EP 12184564
(43) Date of publication of application: 22.07.2015
(73) Proprietor: Janssen Sciences Ireland UC, Little Island, County Cork (IE)
(72) Inventor: BAKKER, Eric, CH-1260 Nyon (CH); BOHETS, Hugo Achiel J., B-2630 Aartselaar (BE); BONROY, Kristien Simonne Raymonda, B-3294 Molenstede (BE); MARCZAK, Marcin Milosz, 1080 Molenbeek-Saint-Jean (BE); ÖZDEMIR, Mahir Sinan, B-St-Amandsberg 9040 (BE); ROYMANS, Dirk André Emmy, B-2300 Turnhout (BE); VANHOUTTE, Koen Jeroom, B-2960 Brecht (BE)
(74) Representative: Daelemans, Frank F.R.
(86) International application number: PCT/EP2013/068991
(87) International publication number: WO 2014/041114

(56) References cited:
- WO-A1-94/07593
- US-A- 4 849 343
- US-A- 5 798 030
- US-A1- 2004 259 073

## Description

The invention relates to a flux based ion selective electrode sensing device for biological molecules or substances. The device comprises an ion selective sensor, an incorporated passive flux of the ion and a, preferably biological, recognition element. The device measures the change in flux of the ion fluxing away from the sensor surface upon binding of the analyte on the sensor surface by means of the biological recognition element.

Potentiometric devices as potentiometric electrodes have a widespread application in fields like chemistry, biology and medicine. The best known example is the well-known pH-meter detecting and/or measuring concentrations of ion species in a test solution. Such electrodes can also be used as sensors detecting analytes including organic acids but also pharmaceutical drugs. Application of said potentiometric devices or electrodes is numerous such as those used in biomedical research, clinical testing, food (contamination) testing or any chemical process control.

At present potentiometric technology capable of detecting proteins is based on variants of pulsed amperometric techniques, in which the potential is recorded after a certain time span. In these cases the ionic flux is generated by applied currents or potentials. Often there is a need for a redox species and/or the presence of auxiliary ion(s) in the analyte solution. Often the presence of an electro conductive polymer film is required herein. The latter limits the shelf life of such a sensor as compared to membrane based organic ion selective sensors used in the current invention. Furthermore in these techniques a potential decay is monitored whereas in the mechanism according to the current invention a potential build-up is monitored until a steady state is observed.

Non-potentiometric ion selective sensors have been reported (WO 94107593 A1 and US 5 798 030 A). In these systems the variation of the ion flux through the membrane is monitored as opposed to the potential over the membrane as in the present invention. Furthermore these sensors react to changes in flux through the membrane and not to fluxes away from the membrane.
Additionally, these sensors rely on another technique, namely impedance spectroscopy, in order to monitor the changes in flux.

Currently there is a high need to develop a universal platform for, preferably disposable, potentiometric immunosensor as "point of care" diagnostic tool for the detection of, for instance, viruses responsible for an infectious disease.

Such disposable diagnostic tools are highly needed for instance in the developing world and/or remote areas.

A recent review of diagnostics for the developing world made it clear that the characteristics of the ideal diagnostic tests are:
- affordable by those at risk of infection,
- sensitive (few false-negatives),
- specific (few false-positives),
- user-friendly and equipment-free (simple to perform and requiring minimal training) and
- rapid (to enable treatment at first visit) and robust (does not require refrigerated storage).

The need for such tests has led to the development of rapid *in vitro* diagnostic (IVD) assays for HIV, RSV, influenza and tropical infections such as dengue, tuberculosis, leptospirosis, melioidosis and malaria.

Rapid diagnosis of infectious diseases has been shown to significantly alter the management of the patient's illness, resulting in a reduction in diagnostic tests performed, reduced antibiotic use, more accurate use of antivirals and better patient management in general. A number of laboratory tests used for the diagnosis of infectious diseases require trained laboratory staff, specialized equipment and are usually time consuming to be used as a viable tool in subsequent treatment options.

Currently, a large majority of the *in-vitro* diagnostic (IVD)-market for the infectious pathogens, is performed by either Enzyme-Linked Immuno Sorbent Assay (ELISA) tests or Polymerase Chain Reaction (PCR) analysis. Because of their complexity, these tests are time consuming and are normally only performed in a laboratory environment. In case of an acute infection the result is not readily available thus leading to loss of precious time before a suitable treatment or therapy can be started.

It has been shown that respiratory syncytial virus (RSV) can cause lower respiratory tract disease (LRD) in infants and patients, for example, after hematopoietic cell transplantation (HCT) and result in substantial early mortality. Early disease detection and intervention, even initiated at a time when the viral load is at its highest, can improve disease outcome in previously healthy, naturally infected children. Indeed, early information about the infecting agent obtained from rapid diagnostic tests has been shown to significantly alter the management of the patient's illness, resulting in a reduction in diagnostic tests performed, reduced antibiotic use, more accurate use of antivirals and better patient management in general.

These existing techniques, however, have already proven their reliability and make accurate diagnosing possible in many cases. The immediate availability of results is preferred and results in reduction of the waiting period and a faster adequate treatment or therapy.

Currently, an increasing number of so-called point of care (POC) diagnostic tests is available on the market. Despite having some great advantages such as being fast, low cost and user friendly, also outside the laboratory, these tests do not always meet the needs of patients and doctors since only a qualitative result is obtained and the limit of detection is not sufficient.

Among these commercialized POC tests, none are however based on the potentiometric technology. Such a technology platform however, has the potential to combine the advantages of the above mentioned laboratory techniques (i.e. reliability, accuracy and low detection limit) with the advantages of the diagnostic POC tests (fast, easy to use and low cost). Modern potentiometry has already provided commercial applications such as the determination of inorganic ions in environmental cases (e.g. F⁻ and NH⁺₄) and clinical settings (e.g. Na⁺, K⁺, Ca²⁺ and Cl⁻). The detection of organic and certainly bio-molecules however has not been explored yet and is obviously of significant commercial and clinical interest. Towards this end, potentiometric sensors of the polymeric membrane type (so-called Ion Selective Electrodes (ISE)) gained considerable interest in the last decade. The potentiometry technology can serve as a technology platform to be a rapid ("dip and read"), low-cost, highly sensitive and user friendly alternative.

The above mentioned technology provides direct detection of antibody-antigen interactions via potentiometry and is obviously a promising research domain.

The current invention is directed towards the development of a (preferably disposable) potentiometric immunosensor as "point of care" diagnostic tool for the detection of, for instance, viruses responsible for an infectious disease. The invention relates to a flux-based ion selective electrode sensing device for biological molecules or substances. The sensing device of the present invention is defined in claim 1. The inventive device measures the change in flux away from the sensor of the ion fluxing out of the sensor upon binding of the analyte on the sensor surface by means of the biological recognition element. This does not limit the detection to biological agents, all analytes that can change the flux (directly or indirectly) can be measured.

In what follows as a "passive flux" is defined as: an ionic flux driven only by difference in concentration. This comprises but is not limited to:
1. A flux of ionic compounds from an inner solution over a membrane to an outer solution. In a particular case this outer solution is the analyte solution. In a more particular case the membrane is an ISE membrane. In a most preferred case the concentration of said ionic compound in the outer solution is approximately zero.
2. A flux of ionic compounds from an inner gel over a membrane to an outer solution. In a particular case this outer solution is the analyte solution. In a more particular case the membrane is an ISE membrane. In a most preferred case the concentration of that ionic compound in the outer solution is approximately zero.
3. A flux of higher ionic concentration from a membrane to a solution. In a particular case this outer solution is the analyte solution. In a more particular case the membrane is an ISE membrane. In a most specific case the concentration of that ionic compound in the outer solution is approximately zero.

Part of the invention is an ion selective membrane which releases (outward flux) the marker ion for which the membrane is selective. Standard ion selective electrodes will limit this outward flux, since it limits the sensitivity of the sensor. It is therefore surprising that an ultra-sensitive sensor can be built by increasing this normally unwanted ionic flux. In principle any ion selective membrane can be used such as but not limiting to: Na⁺, K⁺, Ca²⁺, H⁺, cationic organic molecules or anionic organic molecules.

In order to prevent false positive results on the sensor because of lipophilic ions (e.g. drugs) present in the sample one should use a highly lipophilic ion and/or a very selective ionophore membrane combination. A more preferred ion would have a direct interaction with the analyte such as binding, adsorbing, charging, discharging or decomposing upon interacting with the analyte.

For optimal sensitivity the flux should be such that without binding the concentration of the ion is near to the detection limit of the ISE. This will yield the highest response on binding, due to the logarithmic or possibly supernerstian behavior of ISE's. If more baseline stability is required higher flux rates are advisable. For the specific case of some internal solution ISE's the flux requirements require thin membranes. In order to improve the mechanical stability of such thin membrane a porous support is required.

A further part of the invention is a biological recognition element placed on the analyte side of the ion selective membrane. The analyte of interest is not the ion but the biological substance. This substance is detected due to a change of flux upon binding to the sensor. This change results in the local accumulation of the fluxing ion near the sensor surface which is detected by the membrane that also functions as a potentiometric ISE. Therefore a binding site has to be provided on the top of the sensor membrane.

Another part of the invention is the application of a top layer on the analyte side of the ion selective membrane to prevent aspecific adsorption on the membrane. This layer can consist of a PEG layer (polyethylene glycol) or any other hydrophilic spacer group, BSA (bovine serum albumin) or any other suitable blocker (e.g. a blocking solution, a smart blocker etc).

A preferred embodiment of the invention is a membrane containing a micro porous polypropylene fiber support and a poly vinyl chloride layer comprising N₃ poly vinyl chloride (azide modified poly vinyl chloride, PVC-N3), a plasticizer made of alkylsulphonic acid ester with phenol, and para chloro tetrakis phenyl borate.

In said membrane the poly vinyl chloride layer preferably comprises 25 to 45 % poly vinyl chloride (10%), 30 to 80% plasticizer made of alkylsulphonic acid ester with phenol, and 0.05 to 5% para chloro tetrakis phenyl borate and most preferably 32.5 % polyvinyl chloride (10%), 65.5% plasticizer made of alkylsulphonic acid ester with phenol, and 2% para chloro tetrakis phenyl borate.

Preferably said poly vinyl chloride layer is an azide modified poly vinyl chloride layer.

More preferably said azide modified poly vinyl chloride layer has been also treated with poly ethylene glycol or alternatively with a poly ethylene glycolbiotin linker or a combination of both.

To the invention belongs also the inventive potentiometric sensor or sensing device further comprising a marker (fluxing) ion tetrabutylammonium chloride (TBACI).

Part of the invention is also the method to prepare the membrane according to the invention performing the following steps:
Construction of the membrane:
Body:
- A disk of 6 mm is cut from a micro porous polypropylene fiber support (25 µm thickness, 55% porosity, 0.064 µm pore size).
- The disk is glued to a PVC tube of 6 mm diameter, 1 mm wall size by means of cyclohexanone.
- The tube is then dried overnight.

Thereafter 35 µL of the following electrode cocktail solvent is casted on top of the disk.

Electrode cocktail:
- 0.200 g N3 PVC (10% azide modified [Marcin Pawlak J. Mater. Chem., 2012, 22, 12796]
- 0.400 g Mesamoll,
- 0.012 g Potassium tetrakis(4-chlorophenyl)borate,
- 6 mL THF

After full evaporation of the solvent, derivatisation of the surface is performed by so-called click chemistry [Marcin Pawlak J. Mater. Chem., 2012, 22, 12796]

The two surface modification cocktails are prepared as follows:
PEG
   CuSO₄*5H₂O 46 mg (0.19 mmol) and ascorbic acid 167 mg (0.95 mmol) in 6 mL of water, PEG derivative 5 mg (0.02 mmol) [Marcin Pawlak J. Mater. Chem., 2012, 22, 12796], first dissolved in 0.25 mL THF, then added to the 6 mL H2O.
PEG-B
   CuSO₄*5H₂O 46 mg (0.19 mmol) and ascorbic acid 167 mg (0.95 mmol) in 6 mL of water, biotin-PEG derivative 10 mg (0.02 mmol) [Marcin Pawlak J. Mater. Chem., 2012, 22, 12796], first dissolved in 0.25 mL THF, then added to the 6 mL H2O.

35 µL of this surface modification cocktail is placed on top of the electrode. The electrode is stored in a humid environment over 24 hours.

After this, the electrode is rinsed with DI water. The inside of the tube is filled with 500 µL of a 200 mg TBACI/50 mL DPBS (Dulbecco's Phosphate Buffered Saline) solution. The electrical connection is provided by means of a silver wire. The electrodes are placed on a stirring solution of PBS for 48 hours, prior to measurement.

In addition the use of the above described sensor to measure the concentration of an analyte in a sample solution is part of the invention too.

### Figures

- Figure 1a:: The current sensor design.
- Figure 1b:: Schematic of the sensor close-up.
- Figure 2:: Mechanism of action of the current sensor design.
- Figure 3:: Grid system on ISE
- Figure 4:: QCM data acquired from different type of sensors showing the change in resonance frequency occurring only for the specific electrode (middle) and not for the non-specific electrodes (left and right), supporting the initial sensor design.
- Figure 5:: The data showing the responses of individual sensors from the specific (left) and the control electrodes (right), to a range of RSV concentrations.
- Figure 6:: Calibration curves acquired from the electrodes as in figure 7. The number indicates the total number of electrodes responding to the given concentration of virus (hence less number of electrodes from the control groups)
- Figure 7:: Potentiometric data acquired from specific (peg-b ab-rsv), non-specific (peg-b ab-gp41-negative control) and control (peg ab-rsv and peg ab-gp41) electrodes showing that specific sensors have significantly larger responses to the virus as compared to the control groups.
- Figure 8:: Data showing the correlation between the cycle threshold of the PCR and the viral titer, indicating the sensitivity of the sensor.
- Figure 9:: Data showing the responses of a set of specific (left) and nonspecific (right) electrodes to the varying concentrations of antigen (influenza A2/Aichi/2/68/2)

### Experimental Section

The mechanism of action (MOA) of the current inventive sensor design is based on the flux of a marker ion (TBACI) from the electrode side to the sample side through the membrane and the signal is induced due to the disturbance of this flux upon binding of a target molecule on the sensor surface.

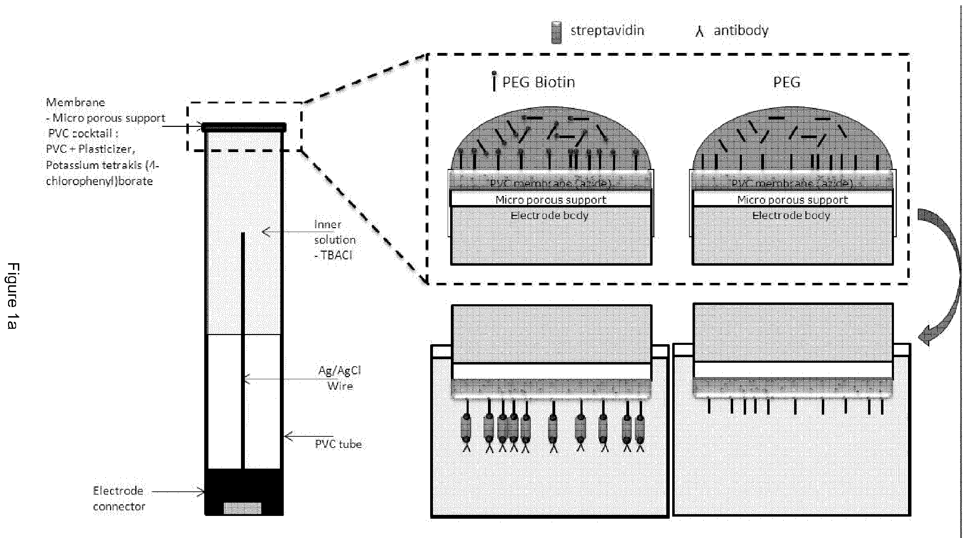

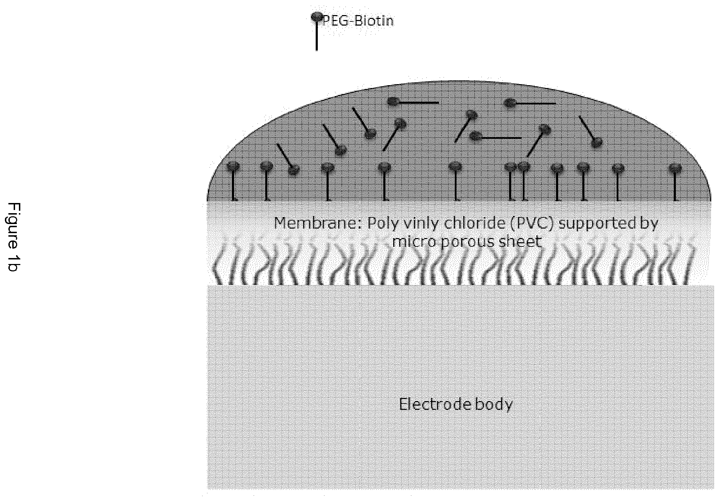

The sensor design and a close-up of the sensor can be seen in the schematic in Figure 1a and Figure 1b, respectively..

A PVC tube of 5.5 cm length and 0.6 cm diameter encases the marker ion solution (inner solution) which through the PVC membrane supported by the micro porous membrane leeches out into the sample side. The electrode is connected to the rest of the electrochemical unit and reference electrode via the Ag/AgCI wire placed in the solution.

Surprisingly, the PVC membrane composition (32.5% PVC-N3 10%, 65.5% Mesamoll, 2% Para Chloro Tetrakis Phenyl Borate) appears to be the most suitable choice. The so called "click chemistry" modification of this PVC membrane is crucial for the correct performance of the membrane and the detection of the analyte in a test sample accordingly. "Click chemistry" is a copper(I)-catalyzed azide-alkyne cycloaddition on a PVC membrane containing partial azide substitutions. The method is described in detail in Pawlak et al. Anal. Chem., 2010, 82, 6887-6894. The membrane contains 32.5% PVC-N3 10%, 65.5% Mesamoll, 2% Para Chloro Tetrakis Phenyl Borate and the membrane is as such made cation selective (ratio solvent cocktail components 1/10 e.g. 100 mg in 1 ml). With a technique called "click chemistry", the azide modified PVC is treated with PEG (Polyethylene glycol) molecules which are very hydrophilic and shield the sensor surface from any interering particle available in the sample. When attached to the PVC surface alone, PEG molecules prevent aspecific adsorption. When modified with Biotin molecule however, the obtained PEG-Biotin (PEG-B) linker can be used to attach the recognition elements on the sensor surface thanks to using streptavidin or avidin as a glue in between (figure 1). Depending on the type of antibody selected, a particular sensor can be made to be specific or non-specific for a given target molecule.

### Electrode membrane surface modification:

- The current set-up allows for working in 4 independent cells. In each cell one can place up to six electrodes and a reference electrode. Each measurement cell was filled with 5 ml PBS and the stirring rate was 150 rpm. After reaching a stable potential (positive drift less than 1 mV/ 10 min) the electrodes were exposed to avidin or streptavidin for 1h under constant stirring at a final concentration of 2.5 µg/ml.
- After each incubation step, the PBS buffer was refreshed. Once a stable baseline potential was established, which usually took up to 30 minutes, electrodes were incubated with biotinylated specific or non-specific antibodies with a final concentration 2.5 µg/ml for 1h. Ab-NUMAX (Motavizumab (proposed INN, trade name Numax) is a humanized monoclonal antibody) and Ab-HIV-1 gp41 were used for the specific and non-specific electrodes, respectively. Before exposing the electrodes to the antigen, the buffer was refreshed once again.

### Virus experiments:

In each measurement cell, electrodes were exposed to four consecutive virus concentrations: 10³, 10⁴, 10⁵ and 5x10⁵ pfu/ml. Time between expositions was kept to be minimum 10 minutes. As a last step, BSA was added in order to compare the electrodes responses and exclude those that were not responsive to BSA.

The proposed mechanism of action, based on the out flux of the marker ion away from the sensor surface, was tested experimentally by using BSA as analyte. In this experiment, different internal solution electrodes were exposed to BSA to test their responses to any possible interaction with this molecule. It should be emphasized that the membrane surface was not made specific by use of any antibody in this case. The membrane was a classical, non-modified PVC membrane.

The mode of action (MOA) is based on the disturbance of the internal marker ion flux upon binding on the sensor surface. As the membrane is very sensitive to the marker ion that leeches out from the internal solution to the sample side where the virus is present, the binding of the virus to the antibodies disturb the marker ion flux away from the sensor surface, changing its local concentration in the vicinity of the sensor. This is picked up by the sensor.

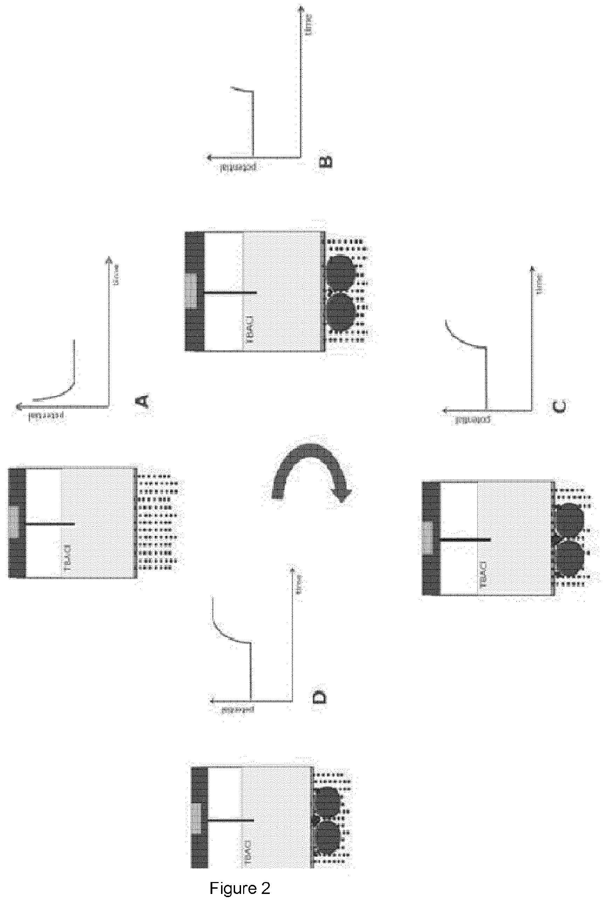

Buildup of a potentiometric signal upon binding is shown below (figure 2):
A) sensor before exposure to the sample (not containing virus) and the internal ion marker leeching out into the sample side, rendering a steady potential baseline,
B) virus introduced to the sample and viral particles bind the Abs on the sensor membrane (read later) hence disturbing the local concentration of the marker ion,
C) The sensor potential continues to rise,
D) The potential levels off as the binding equilibrium is established.

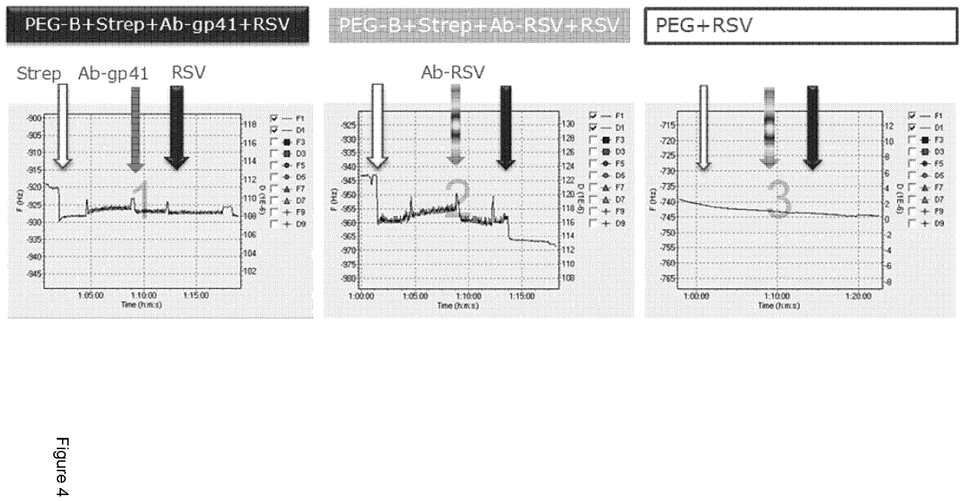

Quartz Crystal Microbalance (QCM) was used to investigate whether the click chemistry modification was executed correctly, rendering the sandwich buildup of the sensor as expected. QCM is a very sensitive balance which indicates a shift from the resonance frequency of the crystal as a result of the binding to the surface. Before the QCM was used, a PVC membrane was used as for a conventional potentiometric sensor onto the gold chips of QCM uniformly using a spin coater. Resulting PVC layer thickness was monitored with Ellipsometry and only those with desired thickness (around 100 nm) were used for the QCM measurements. Figure 4 shows the QCM data acquired from different sensors. The left hand side plot is from an electrode with a nonspecific antibody, which is a control, (Ab-gp41) as used in the potentiometric measurements. The middle is from a specific electrode while the right hand side is from the second type of control containing only PEG linker. As can be seen from this figure 4, a decrease in the resonance frequency was observed only when the electrode used was a specific one. The binding of the Ab and the RSV onto the surface can be clearly seen in the middle graph whereas the baseline signal remains undisturbed for the other sensors. This indicated that the sensor surface was modified as desired.

### Results

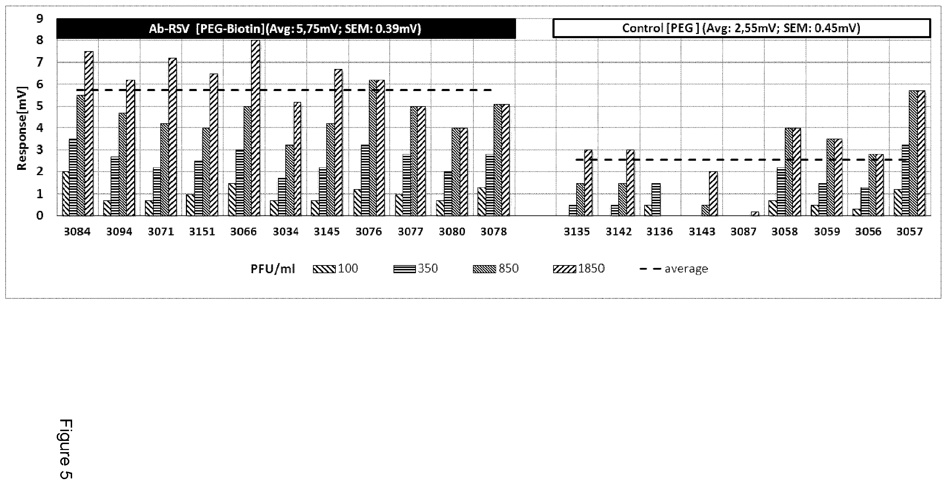

Figure 5 shows the data acquired from specific (left) and control group (right) to a varying RSV concentrations along with the schematic of how each sensor is designed. The specific group made use of the specific Ab against RSV whereas the control group had no Abs and contained PEG linkers only.

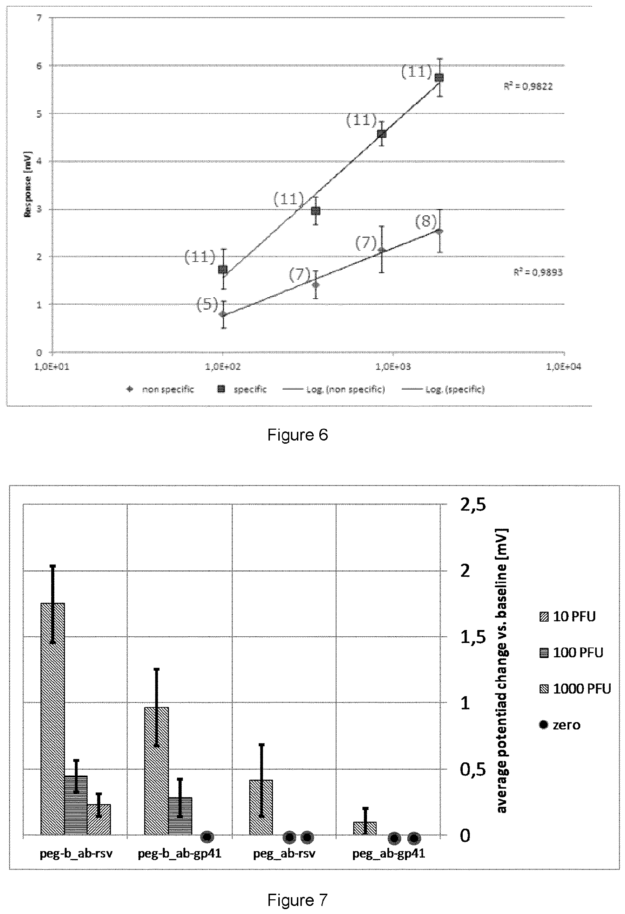

The calibration curves obtained from the same data set is shown in figure 6. It is clear that there is a significant difference between the specific and non-specific electrodes. Numbers in this figure indicate the total number of responding electrodes from each set to a different RSV concentration. As can be seen from the figure, these numbers are systematically less for the control group as the number of electrodes with zero responses is larger than that in the specific group.

Figure 7 shows the data acquired from a larger set including 2 additional types of controls. It should be noted that these measurements were carried out in a flow cell which allows for washing steps and but also conducting measurements without having to remove the electrodes for incubation of a certain agent (e.g. streptavidin).

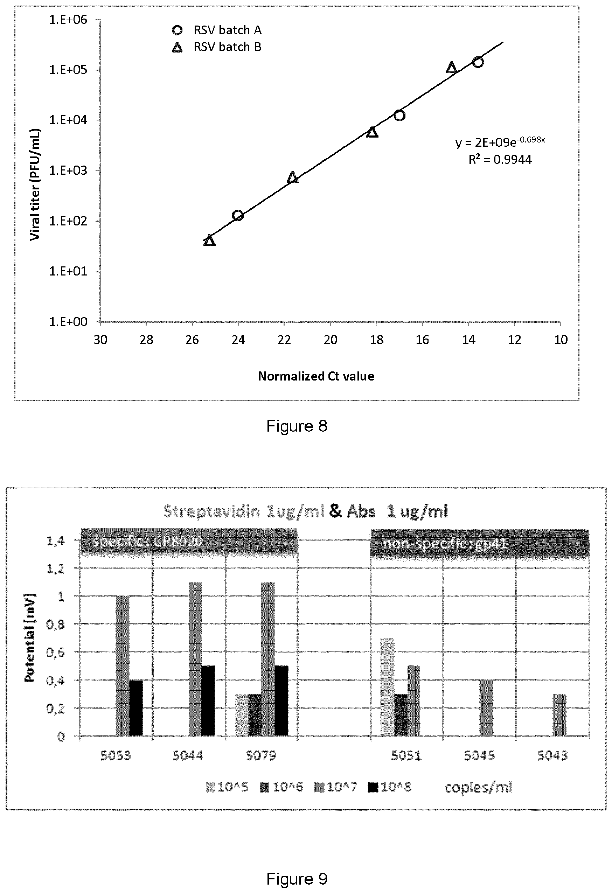

As seen in this graph, only the specific electrodes respond to very low levels of the virus (10 PFU/ml). Two control groups as seen on the left hand-side bar chart do not respond to 10 and 100 PFU/ml RSV injections. To evaluate the true sensitivity of the inventive sensor, the viral concentrations were investigated in batch with an orthogonal technique, quantitative polymerase chain reaction (qPCR). The graph in figure 8 shows the correlation between the cycle threshold of the PCR and the viral titer. Note that Ct values are inversely proportional to the amount of the target nucleic acid in the sample. The sensitivity of the current design is approximately 3 log less than that of the qPCR. This data verifies the sensitivity of the current sensor as compared to the current gold standard of qPCR, which is the corner stone of the modern molecular biology. This fact was also proven by the comparison of the sensor according to the invention with one of the most popular POC RSV tests available on the market. Results showed that Binax test starts responding to the RSV virus at a viral load of about 10⁵ PFU/ml although it seems more sensitive around 10⁶ PFU/ml. Given that the current sensor starts responding to a viral load of about 10³ PFU/ml, it is 10²-10³ times more sensitive than this POC test.

The sensor of the invention was also used to detect influenza virus (A2/Aichi/2/68/2). The sensors were made specific by using specific influenza Abs, whereas HIV-gp41 Abs were used for the non-specific electrodes. A design of experiment was conducted to optimize the Ab and streptavidin concentrations and specific electrodes were found to be more specific to the influenza virus. An example of such entry of the DOE matrix is given in figure 9. When we injected RSV virus to a set of 48 sensors built to detect influenza only, only 4 responded to very high concentration of RSV virus (7,51E+07 cps/ml) showing the specificity of the sensors. The rest of electrodes did show no response at all.

The current sensor design is based on Ion Selective Electrodes (ISE) and as such offers several advantages over other analysis methods. Firstly, the cost of initial setup to make analysis is relatively low. The basic ISE setup includes a meter (capable of reading millivolts), a probe (selective for each analyte of interest).

Due to the double selectivity built in the sensing device of the invention there is a high selectivity. The ISE is not sensitive to non-charged molecules; hence these molecules do not influence the readout. Also non-specific proteins and other colloids are repelled by the blocking agents on the sensor (e.g. PEG) and should not adversely influence the measurements.

Standard well designed ion selective electrodes are insensitive to proteins, turbidity and stirring rate. The well accepted use of these sensors in Na⁺, K⁺ determination in blood analysis is a clear illustration of these facts. If ion selective electrodes are placed in a solution containing 0% of the analyte ion, instability, drift, increased noise is observed and the sensor becomes sensitive to stir rate variation. Therefore in standard ISE use this situation is avoided. In this invention this weakness and the unwanted effects are converted in a flux sensitive detection system that is capable of detecting protein adsorption. Standard electrochemical detection systems require external reagent added to the measurements. In this invention, this step is avoided thanks to the ion flux being released from the sensor naturally. This flux replaces the reagent that is needed for detection of the protein used in standard electrochemical systems.

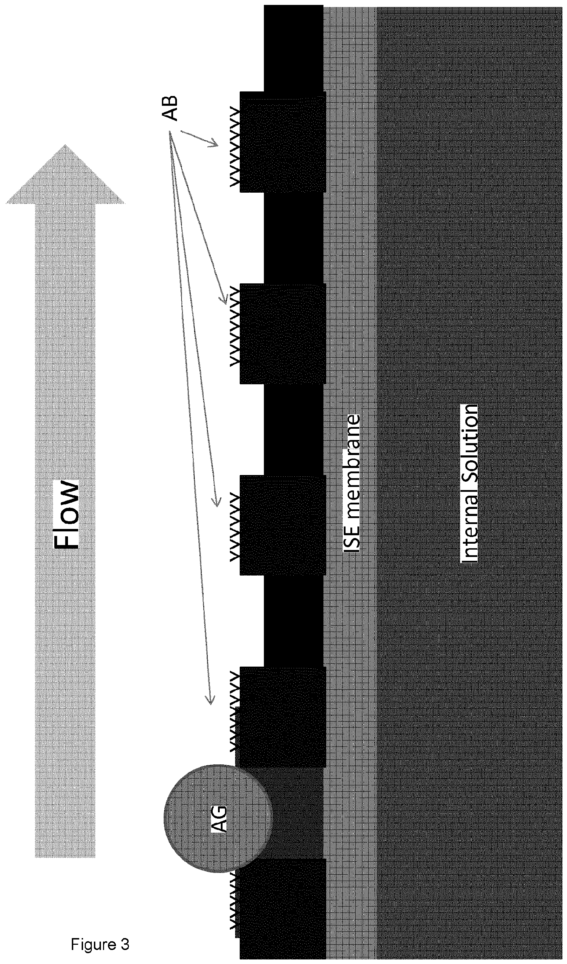

To maximize the performance of the current inventive sensor design the following can be carried out
- Optimization of surface capping:
   ∘ Use of different PEG variations
   ∘ Use of blocking solutions
   ∘ Use of BSA
   ∘ Use of low adsorption plasticizers in the ISE membrane
   ∘ Use of low adsorption polymers in the ISE membrane
- Optimization of flow and surface capping for an optimum blockage/disturbance of the flow; Adaptation of sensor or setup geometry (i.e. full blockage of flow channels by analyte).
- Selecting a marker ion that:
   ∘ Creates selectivity over interfering agents usually present in a test sample and thus protects the electrode from false positive results.
   ∘ Has a specific interaction with the analyte of interest hence increasing flux variation (sensitivity) and selectivity.
   ∘ Exhibits a flux that is more sensitive to the physical blockage by the analyte to the surface hence increasing flux variation (sens itivity).
   ∘ Is highly mobile through the ISE membrane, hence increasing flux and sensitivity. This could for example be done by:
      ▪ A slight modification in the composition of the membrane by adding a calcium-selective ionophore and a calcium nitrate electrolyte as back side solution. This stabilizes the trans-membrane ion flux and hence the signal.
- Surface minimization:
   The inventive sensor principle relies on an achieved surface coverage upon immune reaction. The area of the current sensor is very large (a few millimeters in diameter) compared to, for instance, the 100 nm virus to be detected. Potentiometric microelectrodes with size ranges in the sub-micrometer range are known and could provide a more favorable membrane to virus area than the system described here. Miniaturization of the sensing system to further increase the sensitivity of the sensor and hence lower the limit of detection (LOD) is possible and is within the scope of the current invention.
- Volume minimization
   Measuring in small volumes and/or surface increases signal for the same quantity of analyte since the ISE is concentration and not mass sensitive. Even large sample volumes can be measured in small volume, using the specific adsorption of the analyte on the sensor surface in a flow through setup.
   Placing a grid on top of a sensor could create small cavities that can be specifically blocked by the analyte (Figure 3). The minimal volume created by the analyte sealed grid cavities should result in a fast rise in the local concentration at the ISE membrane. Since the ISE is concentration and not mass sensitive this yields a very high signal and fast response of the sensor. The grid system can also be used in combination with additional forms of volume minimization
- Flow optimization
   Using flow optimization the flux can be tuned to obtain maximal sensitivity on analyte absorption. For optimal sensitivity the flux should be such that without binding the concentration of the ion is near to the detection limit of the ISE. This will yield the highest response on binding, due to the logarithmic behavior of ISE's. If more baseline stability is required higher flux rates are advisable. Furthermore a flow over the sensor can eliminate aspecific adsorption in a similar fashion as is done in quartz crystal microscopy (QCM) and surface plasmon resonance (SPR).

Surface modification and attachment of the recognition element onto the sensor surface can be achieved in a variety of different techniques as follows:
- Binding of the recognition sites (e.g. antibodies, proteins etc.) can be performed by means of nucleophilic groups in the biomolecule (amino groups of lysines, the carboxyl-groups of aspartate and glutamate residues, sulphydryl-groups of cysteines and the imidazole groups of histidine residues.
- Methods for immobilization of the biological receptor molecules are usually adapted from the field of immunochemistry and affinity chromatography.
- A certain combination of a functional group on the surface along with a suitable coupling reagent can be used to target a certain functional group in a certain biomolecule. Many organic derivatives possess different surface functional groups such as amines, thiols and carboxylic acids. These functional groups can be used to form covalent bonds with the functional groups on the target organic molecules such as antibodies or proteins.
- As an alternative, cross linkers can be used to facilitate the formation of a covalent bond. They link one molecule to another. They can be formed by chemical reactions that are initiated when a form of energy is applied on the surface (e.g. heat, pressure or electromagnetic waves).
- Another alternative is to use gold nano-particles to anchor antibodies on the sensor surface. Thiol groups can be used on the surface as sulfur has particular affinity for gold with a binding energy of approximately 20-35 kcal/mol. The thiol group will attach to the gold surface and proteins can then be easily bound to the gold.
- The whole concept of antibodies or proteins as recognition elements can be substituted by molecularly imprinted polymers. In this method, selective recognition sites are formed in synthetic polymers within templates by means of polymerization of monomers in the presence of a templating ligand to avoid using PEG-B, streptavidin and antibodies.
- Instead of antibodies, aptamers specific to the target can be used as recognition element on the sensor.

The invention is not limited to internal solution sensors. Fluxes can also be generated by loading the sensor with the appropriate ion during production, or introducing it in a post-production conditioning step. In this manner sensor systems can be produced according to any of the known ISE's. Such as coated wire electrodes on metal (gold, silver or copper) support, or on conductive supports (glassy carbon, graphite, polythiophene, polypyrrole, polyaniline ...) also gradient based electrodes can be produced (US Patent No: 7,857,962). As a further embodiment introduction of a gel layer between the conductor and the ISE membrane can be inserted for higher stability, more reproducible flux, easy production and prolonged storage.

## Claims

1. A flux based sensing device comprising an ion selective electrode comprising an electrode body and an ion selective membrane that is sensitive to a marker ion, said membrane comprising poly vinyl chloride supported on a microporous support and a recognition element selected from an antibody, an antigen, a molecular imprinted polymer or an aptamer, wherein said polyvinyl chloride comprises 10-40% of an azide modified poly vinyl chloride (PVC-N3), and wherein said electrode body is filled with a solution comprising said marker ion to produce a flux of said marker ion through said membrane.

2. Sensing device of claim 1 comprising a blocking agent wherein the blocking agent is a polyethylene glycol (PEG) unit covalently bound on the PVC-N3 backbone to prevent α-specific adsorption.

3. Sensing device of claim 1 or 2 where the recognition element is bound by a streptavidin-biotinylated PEG unit covalently bound to the PVC-N3 backbone.

4. Sensing device of any of claims 1-3 wherein the micro porous support is a polypropylene fiber support and wherein the poly vinyl chloride layer comprises a plasticizer made of alkyl-sulphonic acid ester with phenol, and para chloro tetrakis phenyl borate.

5. Sensing device of claim 4 wherein said poly vinyl chloride layer comprises 32.5 % N₃ polyvinyl chloride (10%), 65.5% plasticizer made of alkyl-sulphonic acid ester with phenol, and 2% para chloro tetrakis phenyl borate.

6. Sensing device according to any of the claims 1-5 wherein the marker (fluxing) ion is tetrabutylamonium chloride (TBACI).

7. Use of the sensing device of any of claims 1-6 to measure the concentration of an analyte in a sample solution.

## Patentansprüche

1. Sensorvorrichtung auf Flussbasis, umfassend eine ionenselektive Elektrode, die einen Elektrodenkörper und eine ionenselektive Membran umfasst, die für ein Marker-Ion empfindlich ist, wobei die Membran Polyvinylchlorid getragen auf einem mikroporösen Träger und ein Erkennungselement ausgewählt aus einem Antikörper, einem Antigen, einem molekular geprägten Polymer und einem Aptamer umfasst, wobei das Polyvinylchlorid 10-40 % an einem azidmodifizierten Polyvinylchlorid (PVC-N3) umfasst und wobei der Elektrodenkörper mit einer Lösung gefüllt ist, die das Marker-Ion umfasst, um einen Fluss des Marker-Ions durch die Membran zu erzeugen.

2. Sensorvorrichtung gemäß Anspruch 1, umfassend ein Blockiermittel, wobei das Blockiermittel eine Polyethylenglycol(PEG)-Einheit ist, die kovalent an das PVC-N3-Gerüst gebunden ist, um eine spezifische Adsorption zu verhindern.

3. Sensorvorrichtung gemäß Anspruch 1 oder 2, wobei das Erkennungselement durch eine Streptavidinbiotinylierte PEG-Einheit kovalent an das PVC-N3-Gerüst gebunden ist.

4. Sensorvorrichtung gemäß einem der Ansprüche 1-3, wobei der mikroporöse Träger ein Polypropylenfaser-Träger ist und wobei die Polyvinylchloridschicht einen Weichmacher aus Alkylsulfonsäureester mit Phenol und para-Chlortetrakisphenylborat umfasst.

5. Sensorvorrichtung gemäß Anspruch 4, wobei die Polyvinylchloridschicht 32,5 % N₃-Polyvinylchlorid (10 %), 65,5 % Weichmacher aus Alkylsulfonsäureester mit Phenol und 2 % para-Chlortetrakisphenylborat umfasst.

6. Sensorvorrichtung gemäß einem der Ansprüche 1-5, wobei das Marker(Fluß)-Ion Tetrabutylammoniumchlorid (TBACl) ist.

7. Verwendung der Sensorvorrichtung gemäß einem der Ansprüche 1-6 zum Messen der Konzentration eines Analyten in einer Probenlösung.

## Revendications

1. Capteur basé sur un flux comprenant une électrode ioniquement sélective comprenant un corps d'électrode et une membrane ioniquement sélective qui est sensible à un ion marqueur, ladite membrane comprenant du chlorure de polyvinyle supporté sur un support microporeux et un élément de reconnaissance sélectionné parmi un anticorps, un antigène, un polymère à empreinte moléculaire ou un aptamère, dans lequel ledit chlorure de polyvinyle comprend 10 à 40 % d'un chlorure de polyvinyle modifié par un azoture (PVC-N3), et dans lequel ledit corps d'électrode est rempli d'une solution comprenant ledit ion marqueur pour produire un flux dudit ion marqueur à travers ladite membrane.

2. Capteur selon la revendication 1 comprenant un agent de blocage, dans lequel l'agent de blocage est une unité de polyéthylène glycol (PEG) liée de façon covalente sur le squelette de PVC-N3 pour empêcher une adsorption spécifique.

3. Capteur selon la revendication 1 ou 2, dans lequel l'élément de reconnaissance est lié par une unité de streptavidine-PEG biotinylé liée de façon covalente au squelette de PVC-N3.

4. Capteur selon l'une quelconque des revendications 1 à 3, dans lequel le support microporeux est un support en fibre de polypropylène et dans lequel la couche de chlorure de polyvinyle comprend un plastifiant constitué d'un ester d'acide alkylsulfonique avec du phénol, et du parachlorotétrakis(phényl)borate.

5. Capteur selon la revendication 4, dans lequel ladite couche de chlorure de polyvinyle comprend 32,5 % de chlorure de polyvinyle N₃ (10 %), 65,5 % de plastifiant constitué d'un ester d'acide alkylsulfonique avec du phénol, et 2 % de parachlorotétrakis(phényl)borate.

6. Capteur selon l'une quelconque des revendications 1 à 5, dans lequel l'ion marqueur (produisant le flux) est le chlorure de tétrabutylammonium (TBACl).

7. Utilisation du capteur selon l'une quelconque des revendications précédentes 1 à 6 pour mesurer la concentration d'un analyte dans une solution d'échantillon.
